(19) Europäisches Patentamt
European Patent Office
Office européen des brevets

(11) **EP 4 501 266 A1**

(12) **EUROPEAN PATENT APPLICATION**

(43) Date of publication:
**05.02.2025 Bulletin 2025/06**

(21) Application number: **23199022.7**

(22) Date of filing: **22.09.2023**

(51) International Patent Classification (IPC):
**A61B 34/10** *(2016.01)* **A61B 17/34** *(2006.01)*
**A61B 90/00** *(2016.01)* **A61B 18/12** *(2006.01)*

(52) Cooperative Patent Classification (CPC):
**A61B 34/10; A61B 17/3403; A61B 18/12;**
**A61B 18/1477;** A61B 2017/3411; A61B 2034/104;
A61B 2034/2051; A61B 2034/2065; A61B 2090/374;
A61B 2090/376; A61B 2090/3762; A61B 2090/3764;
A61B 2090/378

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **31.07.2023 US 202363529789 P**

(71) Applicant: **Koninklijke Philips N.V.**
**5656 AG Eindhoven (NL)**

(72) Inventors:
• **ISOLA, Alfonso Agatino**
**Eindhoven (NL)**
• **CIANCARELLA, Martina**
**Eindhoven (NL)**
• **HAUTVAST, Guillaume Leopold Theodorus**
**Frederik**
**5656AG Eindhoven (NL)**

(74) Representative: **Philips Intellectual Property &**
**Standards**
**High Tech Campus 52**
**5656 AG Eindhoven (NL)**

(54) **ABLATION APPLICATOR PATTERN MATCHING TO GRID TEMPLATE**

(57) A method (142) for computing applicator insertion patterns for use in grid-based ablation, the method, comprising: at a computing device (14): receiving grid and applicator information (144); computationally iterating an applicator pattern along a plurality of holes in a grid, wherein computationally iterating comprises advancing an applicator pattern object throughout a grid object, wherein the applicator pattern is defined by one or more vertices corresponding to one or more applicator entry trajectories, and an ablation trajectory; and storing pattern position information for a corresponding applicator pattern that matches the holes in the grid, wherein the pattern position information comprises an entry trajectory comprising applicator pattern vertice position or positions and a grid plane normal vector (146).

FIG. 14

**Description**

FIELD OF THE INVENTION

The present invention is generally related to therapy treatment planning, and more particularly, to composite ablation therapy planning.

BACKGROUND OF THE INVENTION

In the field of interventional oncology, percutaneous ablation, including thermal and non-thermal based ablation, is an interventional cancer treatment option. Thermal ablation can be delivered using various ablation modalities, including for example, radiofrequency ablation (RFA), microwave ablation (MWA), high-intensity focused ultrasound (HIFU), focal laser ablation (FLA), and cryo-ablation. Non-thermal ablation techniques include irreversible electroporation (IRE), which uses non-thermal energy to create permanent nanopores in a cell membrane that disrupts cellular homeostasis and ultimately causes cell damage within an applied electric field.

In clinical practice, these ablation procedures consist of placing one or more ablation applicators inside or near a target region with the help of image guidance. Typically, physicians place these needle-like applicators while inspecting real-time ultrasound or interventional radiology images (CT/MR/CBCT), and determine the intended location for the resulting ablation based on information provided from the manufacturer, results in clinical trials, and personal experience. Positioning of the applicators is often determined based on tumor position and size, the device manufacturer's specifications, and the physician's experience. To reduce unwanted damage to nearby healthy tissue, the treatment ablation zone should conform as much as possible to the tumor contours.

Current ablation planning offerings only support regular ellipsoidal ablation zones centered along an axis of a needle-like applicator, while the manufacturer data for a sub-set of the commercially available devices also specify simple (e.g., ellipsoidal) composite ablations, which include ablations achieved by simultaneously ablating with a plurality of applicators positioned in a fixed pattern with respect to each other. The planning of such composite ablations is a complex process, which typically requires applicator positioning in a fixed pattern in 3D space, whilst still enabling full tumor coverage, and sparing of surrounding critical structures. Thus, the clinical users (and automated methods alike) are confronted with a difficult planning problem with huge degrees of freedom and a quite large solution space cardinality. Subsequently, the clinical users face at least one challenge of placing the applicators accurately to achieve the ablation planned. An added challenge is encountered when ablation therapy is delivered using a guiding grid template, since applicator patterns should match grid holes for proper execution. Manually determining possible applicator positioning is a long and arduous process for a clinical user, especially with the complexity involved with multiple vertice insertion patterns.

SUMMARY OF THE INVENTION

One object of the present invention is to improve upon existing systems in the planning of composite ablation zones in ablation therapy and hence the positioning of applicators in a grid. To better address such concerns, in a first aspect of the invention, a method for computing applicator insertion patterns for use in grid-based ablation, the method comprising: at a computing device: receiving grid and applicator information; computationally iterating an applicator pattern along a plurality of holes in a grid, wherein computationally iterating comprises advancing an applicator pattern object throughout a grid object, wherein the applicator pattern is defined by one or more vertices corresponding to one or more applicator entry trajectories, and an ablation trajectory; and storing pattern position information for a corresponding applicator pattern that matches the holes in the grid, wherein the pattern position information comprises an entry trajectory comprising applicator pattern vertice position or positions and a grid plane normal vector. By iterating applicator positions throughout the grid, a plurality of grid matching insertion poses for commissioned applicator insertion patterns, if any, is determined for use in selection of ablation zones for treatment of tumors.

In one embodiment, computationally iterating comprises iteratively advancing, by one hole in a first direction followed by one hole in an orthogonal, second direction, the applicator pattern until all possible applicator patterns in the first and second directions have been attempted. In this search approach, it is assumed that the relative coordinates of the applicators present in a pattern fit the grid spacing over row and column directions.

In one embodiment, computationally iterating comprises, for each hole of the grid: successively anchoring each of the vertices of the applicator pattern to the each hole of the grid; for each applicator pattern position relative to the anchored vertice, determining if all other vertices of the applicator pattern match a respective hole of the grid, wherein the storing the pattern position information comprises storing the pattern position information for each of the applicator pattern positions corresponding to the anchored vertice where the other vertices match the respective holes of the grid; and rejecting applicator pattern positions for a given anchored position where at least one of the vertices does not match a grid hole. In this search approach, no other assumptions are strictly enforced with regard to the grid and applicator pattern geometry when compared to the first approach.

In one embodiment, the method further comprises rotating the applicator pattern a defined angular step and repeating the successively anchoring, determining, storing, and rejecting. The matching via stepped rotations enables a more thorough search of grid hole matches than when the rotation is constrained to ninety-degrees.

In one embodiment, the applicator pattern consists of two vertices, and for a given hole coinciding with one of the two vertices, the computationally iterating comprises: determining a distance between the two vertices; searching for all holes at the distance from the given hole, wherein storing the pattern position information comprises storing pattern position information for all applicator patterns where the two vertices match holes of the grid located at the distance; and repeating the determining and the search for successive holes. This search approach is a modified version of the above-prior approach, where a continuous applicator pattern is implicitly performed rather than angular steps.

In one embodiment, the applicator pattern comprises more than two vertices, and for a given hole coinciding with one of the two vertices, the computationally iterating comprises: determining a distance between the two vertices; searching for all holes at the distance from the given hole and defining each pair of the vertices along the searched distance that match with the holes as a set; for each set, determining remaining applicator pattern vertices, wherein storing the pattern position information comprises storing pattern position information for each set if remaining vertices of the applicator pattern for each set also match holes of the grid; and repeating the determining, searching, and determining for successive holes. This search approach, like the immediately preceding one, applies a continuous applicator pattern that is implicitly performed rather than angular steps, yet for applicator patterns greater than two vertices.

These and other aspects of the invention will be apparent from and elucidated with reference to the embodiment(s) described hereinafter.

BRIEF DESCRIPTION OF THE DRAWINGS

Many aspects of the invention can be better understood with reference to the following drawings, which are diagrammatic. The components in the drawings are not necessarily to scale, emphasis instead being placed upon clearly illustrating the principles of the present invention. Moreover, in the drawings, like reference numerals designate corresponding parts throughout the several views.

Figs. 1A-1C are example representations of complex ablation zones that are represented by certain embodiments of an applicator-grid pattern matching method, in accordance with an embodiment of the invention.

Fig. 2 is schematic diagram that illustrates an embodiment of an example ablation therapy system that facilitates planning, including applicator position planning of an applicator-grid pattern matching method, and guidance to treat a tumor mass or lesion, in accordance with an embodiment of the invention.

Fig. 3 is a schematic diagram that illustrates an applicator pattern comprising a set or group of three applicators arranged in a triangle pattern with a pattern barycenter as the composite ablation entry trajectory origin, in accordance with an embodiment of the invention.

Figs. 4A-4C are schematic diagrams that illustrate example composite ablation zones for two-applicator linear patterns alone and in combination with additional groups of applicators.

Figs. 5(a) and 5(b) are schematic diagrams that illustrate regular and irregular rectangular grids, respectively, that may be used by an applicator-grid pattern matching method, in accordance with an embodiment of the invention.

Figs. 6(a) - 6(d) are schematic diagrams that illustrate example applicator patterns that do and do not match holes of a gird.

Figs. 7(a) - 7(f) are schematic diagrams that illustrate an embodiment of an example first applicator-grid pattern matching method that advances an applicator hole-by-hole according to x-direction and/or y-direction shifts for a two-applicator linear pattern in a grid usage scenario, in accordance with an embodiment of the invention.

Figs. 8(a) - 8(d) are schematic diagrams that illustrate an embodiment of an example second applicator-grid pattern matching method that advances an applicator along a grid, and for each hole, anchors one of the vertices and iterates plural applicator patterns around the anchored hole to determine matches with the unanchored vertices with the grid holes, in accordance with an embodiment of the invention.

Figs. 9A-9B are schematic diagrams that illustrate an example of unmatched applicator patterns and matched applicator patterns, respectively, for the example second applicator-grid pattern matching method, in accordance with an embodiment of the invention.

Figs. 10(a)-10(d) are schematic diagrams that illustrate a rotation used for a currently processed grid hole for the example second applicator-grid pattern matching method to process multiple pattern positions and orientations over the grid plane, in accordance with an embodiment of the invention.

Figs. 11(a)-11(d) are schematic diagrams that illustrate the example second applicator-grid pattern matching method and the neglect or omission of redundant placement patterns anchored at different grid holes, in accordance with an embodiment of the invention.

Fig. 12 is a flow diagram that illustrates an embodiment of the example second applicator-grid pattern matching method, in accordance with an embodiment of the invention.

Figs. 13(a)-13(f) are schematic diagrams that illustrate an embodiment of an example third applicator-grid pattern matching method that modifies the second applicator-grid pattern matching method through use of a continuous applicator pattern match process, in accordance with an embodiment of the invention.

Figs. 14(a)-14(c) are schematic diagrams that illustrate unblended, composite ablations using different applicator and grid patterns, in accordance with an embodiment of the invention.

Fig. 15 is a flow diagram that illustrates an embodiment of an example applicator-grid pattern matching method, in accordance with an embodiment of the invention.

DETAILED DESCRIPTION OF EMBODIMENTS

A cancer diagnosis can be scary for a patient. Aside from fears related to surviving cancer, patients may be worried about treatments and side effects. Historically, surgeries have been open procedures involving large incisions to enable a doctor to directly view the tumor when trying to remove it. Due to advances in technology, a new type of procedure called minimally invasive procedures have emerged as a way to reach the cancer with a smaller incision. This means that patients may experience less pain and go home sooner, and it may mean fewer risks and shorter recovery time.

Because these procedures are less invasive than open surgery, a doctor will not be able to see directly inside the patient's body. He or she may use image guidance, relying on e.g., magnetic resonance imaging (MRI), ultrasound, or other medical imaging to determine a tumor location in the patient. As an example, during an ablation treatment procedure, the doctor may insert into a person a needle that has a tiny laser on the tip, navigate that laser to where the tumor is located using the image guidance, and then proceed to burn off the cancer using the laser. Sometimes multiple needles might be used at the same time to deliver therapy, and in some cases, the placement of the needles might be guided by a grid. Software can be used to develop a plan for placing applicators to try to burn off the cancer while sparing the healthy tissue in a manner not possible by a human, given the complexity of the planning.

Disclosed herein are certain embodiments of an (ablation) applicator-grid pattern matching method and associated system or device (hereinafter, simply also referred to as an applicator-grid pattern matching method) that are used to find a plurality of (e.g., all) possible fixed applicator pattern positions matching grid template holes. For instance, when an ablation treatment is to delivered using a guiding grid template (e.g., a 13x13 brachytherapy grid), a planning algorithm should be able to explore many, if not all, possible applicator pattern positions matching the grid geometry (e.g., identify the set of placement patterns for which all applicator positions (exactly) fit grid holes). Certain embodiments of an applicator-grid pattern matching method generally include three approaches to efficiently determine a set of composite ablation applicator pattern positions matching the grid geometry and usable as input for treatment planning selection (e.g., optimization) and its subsequent execution.

Digressing briefly, existing solutions for applicator pattern placement in conjunction with a grid use manual placement determinations (e.g., by a clinician), where finding possible applicator pattern placements along the grid is a long and tedious process, particularly complicated by applicator patterns with more than two applicators. For instance, not all applicator pattern positions will match with corresponding holes of the grid, and in some instances, positioning and orienting of the applicator patterns may result in applicator entry points outside of the grid. In contrast, certain embodiments of an applicator-grid pattern matching method automatically determines grid hole matching applicator patterns, shortening the applicator pattern placement planning stage and alleviating some of the burdens on clinicians.

Having summarized certain features of an applicator-grid pattern matching method of the present disclosure, reference will now be made in detail to the description of an applicator-grid pattern matching method as illustrated in the drawings. While an applicator-grid pattern matching method will be described in connection with these drawings, there is no intent to limit it to the embodiment or embodiments disclosed herein. Further, although the description identifies or describes specifics of one or more embodiments, such specifics are not necessarily part of every embodiment, nor are all of any various stated advantages necessarily associated with a single embodiment. On the contrary, the intent is to cover alternatives, modifications and equivalents included within the principles and scope of the disclosure as defined by the appended claims. For instance, two or more embodiments may be interchanged or combined in any combination. Further, it should be appreciated in the context of the present disclosure that the claims are not necessarily limited to the particular embodiments set out in the description.

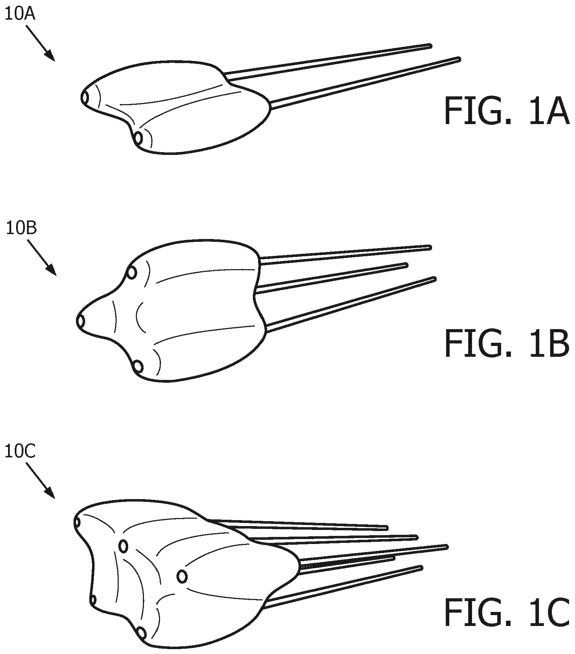

Although the focus of the present disclosure is on the auto-placement of (e.g., all) possible positions (including orientations) of a commissioned applicator set (e.g., comprising one or more applicators corresponding to an ablation trajectory) within a grid, some background on ablations resulting from placement and selection according to an ablation treatment plan is described briefly below. Existing ablation planning methods are typically based on manufacturer data on the expected ellipsoidal ablation zone achieved using an ablation device using plural combinations of power and duration. In the case of composite ablations, manufacturer data on the expected ellipsoidal, composite ablation zones uses a combination of spacing between applicators, power, and duration. Such composite ablation zones are rigidly based on a fixed pattern of the applicators, where each applicator is treated as an independent entity. Publications exist that also describe or represent composite ablation zones using a simple binary union of ellipsoids, though such techniques would not be usable for microwave (MW) and irreversible electroporation (IRE) devices, which deliver complex ablation zones with non-ellipsoidal shapes. In the market, different probe models (probe and applicator used interchangeably herein) are given with datasheets indicating the expected geometry of both each single ablation zone delivered by a given probe configuration when used in isolation, and also the expected composite ablation zone delivered by different geometrical probe configurations. In some devices, the expected composite ablation zone has an ellipsoidal shape, however there are also other devices, for instance for MWA or IRE treatment, where the composite ablation zone can have quite complex shapes produced as a blending of the multiple ellipsoids delivered by each probe, as depicted by the example complex composite ablation zones 10A-10C in Figs. 1A-1C, respectively.

In the description that follows, the ablations resulting from execution of the planned applicator placements, the planning involving the employment of an applicator-grid pattern matching method and selection (e.g., optimization) methods, may be in the form of regular ablation zone shapes (e.g., ellipsoids, cylinders, etc.) centered along the axis of an applicator, and/or composite ablations based on plural applicators positioned in a fixed pattern with respect to each other or as configured by a user. Further, for the composite ablations resulting from the aforementioned planning, the ablation zone computations may be based on in-vivo or ex-vivo results, a binary union of ellipsoids, or based on the blending of implicit functions to represent composite ablation zones.

Note that reference herein to a composite ablation zone refers to the expected total ablation zone shape delivered by a set or group of executed applicators. Though a focus of the present disclosure is on the planning stage, and in particular, grid-based applicator positioning to ultimately obtain composite ablations, the applicator-grid pattern matching method may be used for planning (and eventual execution of) non-composite ablation zones as well.

Note that reference to positioning or placement of the applicator pattern along the grid, when describing certain embodiments of an applicator-grid pattern matching method, is referring to the positioning (including orienting, such as via stepped or continuous rotations) of computational objects (e.g., in an object-oriented programming or logic space) of the grid and applicator patterns. Also, reference to an applicator pattern (the object) is intended to include a single or multiple applicators arranged to deliver an ablation trajectory, the applicator arrangement defined by applicator trajectories or vertices relative to the ablation trajectory and aligning or matching with holes along the grid.

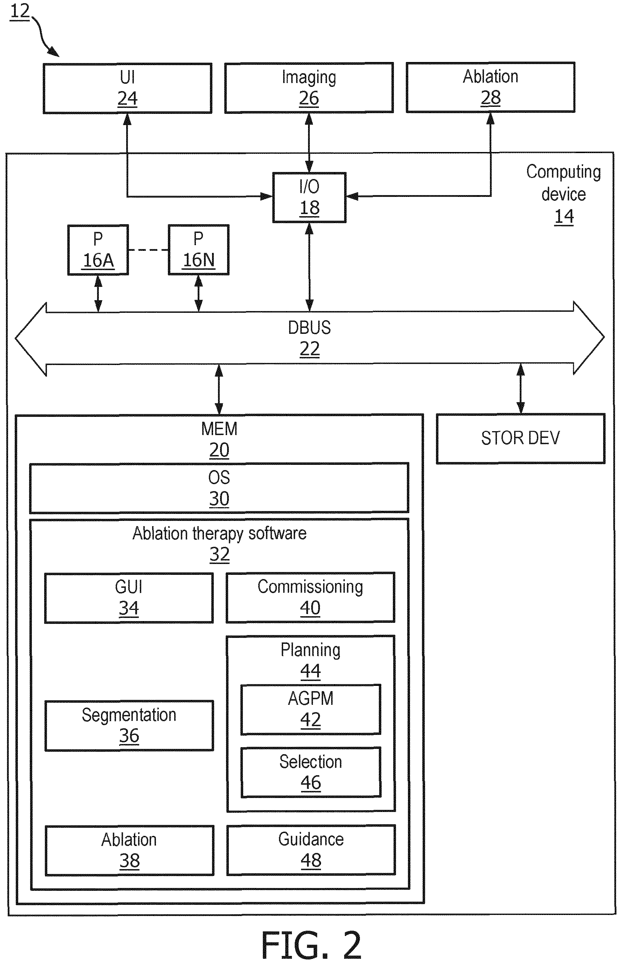

Fig. 2 illustrates an ablation therapy system 12 that facilitates the planning and guidance of one or more ablation protocols to guide treatment of a target region (e.g., a tumor mass or lesion, including any margin) in a subject, and also includes applicator pattern positioning functionality of an applicator-grid pattern matching method. Note that reference herein is made to the ablation therapy system 12 responsible for treatment based on the planning and guidance functionality, though in some embodiments, the planning, guidance, and/or treatment may be implemented as separate systems in communication or cooperation with each other. Successful treatment of large tumors can be achieved by planning ablation probe positions with the intent that no part of the tumor is left untreated, and accurately executing the plan. In general, the ablation therapy system 12 provides for automated and interactive planning of ablation therapy, guidance of applicator placement, and ablation therapy treatment based on the planning and guidance. The ablation plan ensures that a majority of (or potentially all) areas of the tumor are covered, and reports the number of ablations involved for an ablation treatment using a particular probe or group(s) of probes. The ablation therapy system 12 also utilizes selection techniques to minimize the number of ablations. Since the plan is quantitative, it can be carried out using a robot and/or by using registered image guidance, such as by quantitatively tracking the ablation probe.

In the depicted embodiment, functionality of the ablation therapy system 12 is implemented as a system of co-located software and hardware components collectively embodied as a computing device 14 (which may include a medical device) and plural sub-systems, wherein the computing device 14 is operatively connected to the plural sub-systems, as described below. In some embodiments, the computing device functionality may be embedded in one of the sub-systems, or one or more of the herein-described sub-system functionality may be integrated into fewer devices. It should be appreciated that, in some embodiments, functionality of the ablation therapy system 12 may be implemented via plural computing devices that are networked at a similar location or situated in different locations, potentially remote from each other, and connected via one or more networks. The computing device 14 includes one or more processors 16 (e.g., 16A...16N), input/output interface(s) 18, and memory 20, etc. coupled to one or more data busses, such as data bus 22.

The processor(s) 16 may be embodied as a custom-made or commercially available processor, including a single or multi-core central processing unit (CPU), tensor processing unit (TPU), graphics processing unit (GPU), vector processing unit (VPU), or an auxiliary processor among several processors, a semiconductor-based microprocessor (in the form of a microchip), a macroprocessor, one or more application specific integrated circuits (ASICs), field programmable gate arrays (FPGUs), a plurality of suitably configured digital logic gates, and/or other existing electrical configurations comprising discrete elements both individually and in various combinations to coordinate the overall operation of the computing device 14.

The I/O interfaces 18 comprise hardware and/or software to provide one or more interfaces to various sub-systems, including to one or more user interface(s) 24, an imaging sub-system 26, and an ablation sub-system 28. The I/O interfaces 18 may also include additional functionality, including a communications interface for network-based communications. For instance, the I/O interfaces 18 may include a cable and/or cellular modem, and/or establish communications with other devices or systems via an Ethernet connection, hybrid/fiber coaxial (HFC), copper cabling (e.g., digital subscriber line (DSL), asymmetric DSL, etc.), using one or more of various communication protocols (e.g., TCP/IP, UDP, etc.). In general, the I/O interfaces 18, in cooperation with a communications module (not shown), comprises suitable hardware to enable communication of information via PSTN (Public Switched Telephone Networks), POTS, Integrated Services Digital Network (ISDN), Ethernet, Fiber, DSL/ADSL, Wi-Fi, cellular (e.g., 3G, 4G, 5G, Global System for Mobile Communications (GSM), General Packet Radio Service (GPRS), etc.), Bluetooth, near field communications (NFC), Zigbee, among others, using TCP/IP, UDP, HTTP, DSL.

The user interface(s) 24 may include a keyboard, scroll-wheel, mouse, microphone, immersive head set, display device(s), etc., which enable input and/or output by or to a user, and/or visualization to a user. In some embodiments, the user interface(s) 24 may cooperate with associated software to enable augmented reality or virtual reality. The user interface(s) 24, when comprising a display device, enables the display of segmented anatomical structure(s), including tumor(s), and the virtual (e.g., simulated) and actual placement of applicators and identification of ablation zones and ablation zone coverage, as well as virtual bounding boxes (e.g., grid objects). In some embodiments, the user interface(s) 24 may be coupled directly to the data bus 22.

The imaging sub-system 26 includes one or more imaging sub-systems and/or image storage sub-systems that are used to enable visualization of tumors, virtual or actual applicator placement, and/or single or composite ablation zones during pre- and per-operative (e.g., during actual applicator placement and/or ablation treatment) imaging. The imaging sub-system 26 may include ultrasound imaging (e.g., 2D/3D in real-time), fluoroscopy (real-time), magnetic resonance (e.g., MR, in 2D/3D real-time or static), computed tomography (e.g., CT (3D static)), cone beam CT (e.g., CBCT (3D static)), single photon emission CT (SPECT), and/or positron emission tomography (PET). In some embodiments, the images may be retrieved from a picture archiving and communication systems (PACS) or any other suitable imaging component or delivery system.

The ablation sub-system 28 may include any one of various ablation modalities, including radiofrequency ablation (RFA), microwave ablation (MWA), high-intensity focused ultrasound (HIFU), focal laser ablation (FLA), irreversible electroporation (IRE), and cryo-ablation. In some embodiments, software in memory 20 may be used to interface with the ablation sub-system 28 to adjust settings (e.g., probe distance, power, duration), track positioning of the applicators, and/or in the case of robotic-based ablation therapy, control the actual placement and energizing of the applicators.

The memory 20 may include any one or a combination of volatile memory elements (e.g., random-access memory RAM, such as DRAM, and SRAM, etc.) and nonvolatile memory elements (e.g., ROM, Flash, solid state, EPROM, EEPROM, hard drive, tape, CDROM, etc.). The memory 20 may store a native operating system, one or more native applications, emulation systems, or emulated applications for any of a variety of operating systems and/or emulated hardware platforms, emulated operating systems, etc. In some embodiments, a separate storage device (STOR DEV) may be coupled to the data bus 22 or as a network-connected device (or devices) via the I/O interfaces 18 and one or more networks. The storage device may be embodied as persistent memory (e.g., optical, magnetic, and/or semiconductor memory and associated drives). In some embodiments, the storage device or memory 20 may store manufacturer data pertaining to various applicators and applicator patterns.

In the embodiment depicted in Fig. 2, the memory 20 comprises an operating system 30 (OS) (e.g., LINUX, macOS, Windows, etc.), and ablation therapy software 32. In one embodiment, the ablation therapy software 32 comprises a plurality of modules (e.g., executable code) co-hosted on the computing device 14, though in some embodiments, the modules may be distributed across various systems or sub-systems over one or more networks. The ablation therapy software 32 comprises a graphical user interface (GUI) module 34, a segmentation module 36, and an ablation module 38. The ablation therapy software 32 also comprises a commissioning module 40, and a planning module 44, which in one embodiment comprises an applicator-grid pattern matching (AGPM) module 42 and a selection module 46. The commissioning module 40 may include additional functionality, such as implicit functions or algorithms for blending of composite ablation zones, ellipsoid union algorithms, or manufacturing data on expected composite (and single) ablations. In some embodiments, such information may be stored elsewhere (e.g., at the ablation module 38). The ablation therapy software 32 further comprises a guidance module 48, which through cooperation with the user interface(s) 24, imaging sub-system 26, and ablation sub-system 28, among other modules, guides placement of the applicators via overlay graphics on top of images. Note that functionality of the modules of the ablation therapy software 32 may be shared amongst each other. In some embodiments, there may be fewer or additional modules. For instance, functionality of some modules may be combined, or additional functionality may be implemented yet not shown, such as a communication module, security module, etc.

Functionality of one or more of the various modules is briefly explained here, with further description below. The GUI module 34 provides for reconstruction and rendering on the user interface(s) 24 (e.g., a display device) of image data received from imaging sub-system 26. Objects such as a lesion, organs, critical and/or healthy anatomical structures (or generally, regions not intended for ablation), may be segmented automatically using existing algorithms or by hand with drawing tools along the axes via the segmentation module 36. The segmentation module 36 produces a description of the volumetric regions associated with the specific objects according to existing methods. The GUI module 34 may also provide visualizations of ablation zones, applicator patterns, and applicator pattern placement for application to one or more target regions, whether virtually placed (e.g., pre-operative) or actually placed (during per-operative implementations).

The ablation module 38, in conjunction with the GUI module 34, provides for the display of user-selectable options for applicators and/or applicator zones. For instance, the ablation module 38 may optionally (e.g., in addition to or in lieu of being stored in the commissioning module 40 or elsewhere) store manufacturer data on expected ellipsoidal ablation zones based on different settings (e.g., power, duration, probe separation distance, etc.) for the ablation sub-system 28, and/or pre-defined (e.g., from manufacturer data) applicator patterns (e.g., single probe, two probe, three probe, etc.). In some embodiments, the ablation module 38 may track positioning of the applicators of the ablation sub-system 28 using electromagnetic or other types of sensors on or near the applicators, and/or use image analysis for tracking. The ablation module 38 controls applicators during ablation procedures based on the settings, and in some embodiments, may be used in conjunction with robotic controls to control the placement and activation of the applicators.

The commissioning module 40, in cooperation with the GUI module 34, enables user-configured applicators and/or ablation zones and/or, selection of pre-defined applicator patterns. The commissioning module 40 may also include an implicit functions module (not shown), which may be used to support non-ellipsoidal composite ablation zones based on blending of ellipsoidal zones from the applicators of a group (an applicator pattern). The commissioning module 40 may be used for a grid or grid-less environment. The commissioning module 40 may provide for fixed positioning (as prescribed by manufacturer data obtained, in one embodiment, by the ablation module 38) or as configured by a user (e.g., clinical user) via the GUI module 34.

The planning module 44 provides for the automated and/or interactive planning of single and composite ablations. For instance, based on segmented anatomical regions, the planning module 44 utilizes the commissioned data to precompute a plurality of (e.g., in some embodiments, all possible) probe or applicator configurations from which a preferred solution or plan is selected. For the grid-based planning, the planning module 44 uses the applicator-grid pattern matching module 42, which includes algorithms for computationally iterating an applicator pattern object within a grid object to determine possible positions, including orientations, where the applicator pattern matches the holes of the grid. In one embodiment, the applicator-grid pattern matching module 42 comprises three different algorithms for searching and/or determining (e.g., all) possible pattern positions that match the holes of the grid, where those applicator patterns are provided to the selection module 46 for selection for eventual treatment/therapy. In one embodiment, the algorithms comprise a first matching algorithm where matching is performed by shifting the applicator patterns along all rows and columns of the grid. The second matching algorithm includes matching using discrete local points sets matching. The third matching algorithm includes matching using continuous local points sets matching. Further description of these algorithms/methods is set forth below.

The planning module 44 further comprises the selection module 46, which analyzes information associated with the tumor(s) and applicator pattern positioning and ablation zones, and defines a set of ablation positions with orientations. In one embodiment, the selection module 46 comprises existing optimization functionality, which identifies the fewest (or fewer) number of ablations possible that cover the tumor region (e.g., tumor or tumor plus margin). In some embodiments, the selection module 46 identifies the ablation positions with orientations that spare the healthy tissue (e.g., that minimizes or reduces the risk of collateral damage). In some embodiments, additional object volumes are segmented that denote critical regions of tissue or bone that are not to be ablated, and the selection module 46 attempts to generate either the fewest ablations or minimize collateral damage, while also avoiding or reducing the risk of ablation of these regions. In some cases however, the selection module 46 produces unablated areas, whereupon the user is alerted and the regions can be displayed on the user interface(s) 24.

The guidance module 48 provides for user interactive guidance of applicator placement per the planned positions and selection, as described below.

A general description of example operations of the ablation therapy software 32 is described below. After tumor and risk organ segmentation via segmentation module 36, the commissioning module 40 is used to enable commissioning under the direction or instruction of a user (e.g., clinical expert) via GUI module 34. For instance, during commissioning, the clinical expert decides which probes and probe settings to use. Probe setting may include probe model, geometry entry pattern (e.g., single probe, two probes in a linear pattern, three probes in a linear or triangular pattern, four probes in a square or rectangular pattern, etc.), and expected ablation zone (e.g., ellipsoid or complex blended shape). In one embodiment, the ablation therapy software 32 may provide a commissioning GUI (e.g., via GUI module 34) for interaction with a clinical expert for complex blended shapes, where blending parameters may be manually tested, or in some instances, these blending parameters may be automatically computed if the user loads meshes or binary volumes representing an expected composite ablation zone per manufacturer data.

Once commissioning is completed, operation by the ablation therapy software 32 resumes with the planning module 44. In grid scenarios, the applicator-grid pattern matching module 42 may computationally iterate a commissioned applicator pattern (object) throughout the grid (object) using one of the matching methods, and pass this information to the selection module 46. In one embodiment, the selection module 46 uses the information passed by the applicator-grid pattern matching module 42 and other commissioned data to precompute possible probe configurations from which a preferred solution or plan for treating a target region is selected. For instance, the selection module 46 may determine which of the blended (or in some embodiments, unblended and/or single) ablation zones are to be selected or removed. As an illustrative example, and in the case of the selection module 46 comprising optimization functionality, an iterative solver of the selection module 46 may select a plan out of a plurality of (and in some embodiments, all) possible precomputed probe configurations (e.g., including single and/or multi-probe groups delivering a simple ellipsoid and/or blended or unblended ablation zones, respectively), such as a single probe delivering an ellipsoid ablation in one area of the target region and a triangular group of probes delivering a composite blended ablation at another area of the target region.

In one embodiment, the selection module 46 comprises a set-covering, greedy, iterative optimizer that selects a preferred set of probe groups (e.g., triangle patterns or other geometric patterns including squares, rectangles, dots, etc.) to fully cover the tumor (target region) while sparing the at-risk organs nearby. Note that selection of the preferred set may be an optimized or best set in some embodiments, or a set that is less than what may be considered as the best set yet suitably meets certain criteria. For instance, example criteria may include quantity or computational time of iterations in selection, anticipated time of treatment, the quantity, size, and/or maturity of the tumors, assessed or predicted risk of malignancy, confidence in such an assessment or prediction, subject and/or subject family history, among other factors established in the medical community and/or by the clinical expert. One or more of these factors may be considered and/or weighted relative to one or more other factors in a manner that might result in an outcome that is less than the best outcome as achieved by an optimizing function, yet preferred based on the established criteria. In some embodiments, a preferred solution may comprise greater subjectivity in considering an outcome than an optimization function, and/or in some embodiments, an optimizing function may be used yet constrained based on computational resources and/or computation time.

Explaining further, and in the particular case in which a grid template is used, the selection module 46 uses the possible pattern placements determined by the applicator-grid pattern matching module 42 to select (e.g., where selection may be achieved via an optimization algorithm in some embodiments) the applicator patterns (and hence sets of applicator groups) to be used for therapy/treatment. Following the planning stage, operations resume with the guidance module 48 for guided physical placement of the probes, and then actual execution of ablation therapy.

Note that the memory 20 and storage device may each be referred to herein as a non-transitory, computer readable storage medium or the like.

Execution of the ablation therapy software 32 may be implemented by the one or more processors 16 under the management and/or control of the operating system 30.

When certain embodiments of the computing device 14 are implemented at least in part with software (including firmware), it should be noted that the ablation therapy software 32 (and its corresponding components) can be stored on a variety of non-transitory computer-readable (storage) medium for use by, or in connection with, a variety of computer-related systems or methods. In the context of this document, a computer-readable medium may comprise an electronic, magnetic, optical, or other physical device or apparatus that may contain or store a computer program (e.g., executable code or instructions) for use by or in connection with a computer-related system or method. The software may be embedded in a variety of computer-readable mediums for use by, or in connection with, an instruction execution system, apparatus, or device, such as a computer-based system, processor-containing system, or other system that can fetch the instructions from the instruction execution system, apparatus, or device and execute the instructions.

When certain embodiments of the computing device 14 are implemented at least in part with hardware, such functionality may be implemented with any or a combination of the following technologies, which are all already existing in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), TPUs, GPUs, and/or other accelerators/co-processors, etc.

One having ordinary skill in the art should appreciate in the context of the present disclosure that the example computing device 14 is merely illustrative of one embodiment, and that some embodiments of computing devices may comprise fewer or additional components, and/or some of the functionality associated with the various components depicted in Fig. 2 may be combined, or further distributed among additional modules or computing devices, in some embodiments. It should be appreciated that certain well-known components of computer systems are omitted here to avoid obfuscating more relevant features of the computing device 14.

Before commencing further particulars of certain embodiments of the applicator-grid pattern matching module 42, a brief description follows about certain requirements to fulfill when delivering a composite ablation zone as described on vendor datasheets. The definition or representation of composite ablation zones, also referred to as commissioning, prescribes the position and orientation of the applicators (or equivalently, probes) relative to the ablation zone, as well as the dimensions of the ablation zone. This commissioning may be performed as fixed (e.g., performed according to the manufacturer of the system) or configurable (e.g., performed by end-users of the system).

When configurable by the end-user, the system is equipped with a graphical user interface (GUI) (e.g., via GUI module 34, Fig. 2) that enables specifying the commissioning information, including the relative positioning of applicators and the composite ablation zone (e.g., tip offsets, spacing, etc.) and/or ablation zone dimensions (e.g., major and minor axes of an ellipsoid). The relative positioning of applicators and ablation zones can be expressed with respect to the center of the ablation zone or the tip location of any of the associated applicators. Further, the GUI is used to enable a user to virtually position a single probe or a group of probes that have been chosen during the commissioning phase, with feedback of the resulting ablation zones (e.g., composite or otherwise) presented on-the-fly to the user.

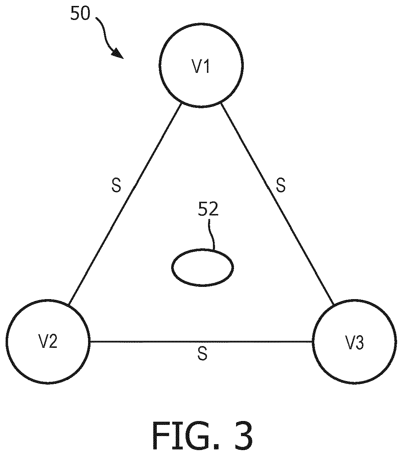

The user may be supported to specify composite ablation zones by a GUI (e.g., via GUI module 34 in conjunction with user interface(s) 24 as in Fig. 2) providing a CAD-like drawing environment enabling the relative positioning of applicators and composite ablation zone or a selection of pre-defined placement patterns, for which a limited number of parameters enables the relative positioning of applicators and composite ablation zone. The pre-defined placement patterns replicate information provided by the ablation device manufacturer, which includes, but is not limited to, an applicator pattern comprising a line with applicators separated by distance, s (2 applicators), a triangle with sides of length s (3 applicators), a square with sides of length s (4 applicators), a trapezoid specified by a length and angle (4 applicators), etc. When the user selects one of these patterns, and provides values for the associated parameter(s), the system computes the relative positioning of the applicators with respect to the center of the ablation zone. For instance, and referring to Fig. 3, shown is a group of three applicators in the form of a triangle pattern 50. The three vertices V1, V2, and V3 represent respective applicator entry points (corresponding to entry trajectories), and the triangle pattern 50 comprises a composite ablation zone entry trajectory 52 (or simply, ablation trajectory) corresponding to the triangle barycenter. Parameter s indicates the length of each triangle side.

As an example, for an insertion pattern with an equilateral triangle shape of side s, the ablation entry trajectory origin may be positioned at (x,y) = (0,0) and the origins of the three applicator entry trajectories (i.e., triangle vertices V1, V2 and V3) may be obtained as follows:

$$V1(x,y) = \left( -\frac{s}{2}, -\frac{1}{3}\sqrt{s^2 - (\frac{s}{2})^2} \right)$$

EQN. 1

$$V2(x,y) = \left( \frac{s}{2}, -\frac{1}{3}\sqrt{s^2 - (\frac{s}{2})^2} \right)$$

EQN. 2

$$V3(x,y) = \left( 0, \frac{2}{3}\sqrt{s^2 - (\frac{s}{2})^2} \right)$$

EQN. 3

As described above, the ablation therapy system 12 (Fig. 2) may support not only ellipsoidal but also non-ellipsoidal composite ablation zones, such as those achieved by IRE, by providing a commissioning GUI (e.g., a GUI rendered on a display device, such as via GUI module 34 and user interface(s) 24 in conjunction with commissioning module 40, as in Fig. 2) that enables describing a composite ablation zone as a combination of ellipsoids along with an additional blending parameter (e.g., via an implicit functions module in, for instance, the commissioning module 40 (Fig. 2)). The blending of individual ellipsoids into a composite ablation zone may be based on the mathematical concept of implicit functions, as provided via algorithms in the implicit functions module of the commissioning module 40. An implicit function F(P) representing a surface specifies a position P to be on the surface if F(P) = 0. If the position is inside or outside the surface, F(P) < 0 or F(P) > 0, respectively. For an ellipsoidal surface around centroid Pc = (xc, yc, zc), the implicit function is defined by:

$$F(P) = F(x,y,z) = \frac{x - x_c}{r_x^2} + \frac{y - y_c}{r_y^2} + \frac{z - z_c}{r_z^2} - 1$$

EQN. 4

To calculate the composite ablation, the implicit functions of Ns individual ablation zones may be combined via another implicit function:

$$H(P) = 1 - \sum_{i=0}^{N_s} g(F_i(P)) = 0$$

EQN: 5

Here, the blending function $g(x)$ can be defined in various ways, including, but not limited to:

$$g(x) = e^{-bx}$$

EQN: 6

$$g(x) = \frac{a}{R^{n+1}} (x - R)^n (\frac{R}{a} - x))$$,

EQN: 7

where b, a, R, and n are blending parameters, and where these two blending functions are used to enforce a smooth transition between two basic surfaces (e.g., two ellipsoids). The exponential blending function of equation 6 comprises parameter b, where b > 0 is a parameter to control the blending curve steepness (e.g., where b = 1 comprises two separated ellipses, b = 0.5 comprises slight blending, somewhat similar to an hourglass shape, and b = 0.3 comprises more extensive blending where the overall shape is more oblong with a slightly narrower middle region). Equation 7 comprises a distance-based blending function, where the function is true if x ≤ R, otherwise, g(x) = 0. In equation 7, a ∈ [0,1], n ∈ N*, and R > 0 is a blending radius. Here, the blending action vanishes when x > R, and parameter n adjusts the blending function by steps and parameter, a, realizes a fine-tuning in between the steps. The value(s) for the blending parameter(s) may be set directly by a value provided by the user, or indirectly by automatic adjustment of the blending parameter to mimic a composite ablation shape prescribed by a mesh provided by the user. For instance, the user may change one or more of the blending parameter values, and the resulting blended shape may be computed on-the-fly and the commissioning module 40 may, in cooperation with the GUI module 34, provide visual feedback of the newly blended shape. In some embodiments, the computed shape may be plotted next to the shape proposed by the manufacturer. In some embodiments, quality metrics may be provided (e.g., Dice coefficient) to show how the current blended shape overlaps with a reference shape. As to the mesh-based value determination approach, in instances where an ablation device manufacturer computes an expected composite ablation shape, such as via the use of bio-heat transfer modeling the probes and the anatomy, these computed composite ablation regions may be provided as meshes or binary masks. In such instances, blending function parameters may be inferred using, say, an I-squared optimization method or other methods that minimize or reduce the error between the computed meshes and a reference ground truth mesh provided by the manufacturer.

As explained above, the ablation therapy system 12 include an automatic planning component (e.g., via planning module 44, Fig. 2) to support a user (e.g., clinical expert) in establishing an ablation plan. The ablation planning selection problem generally consists of a preferred (e.g., depending on one or more user criteria), or in some embodiments, optimal selection of a set of applicators delivering single/regular and/or composite ablation zones to ablate the target region (e.g., tumor plus an optional margin) as well as possible (e.g., at 100% of its volume) while, at the same time, sparing nearby organs at risk and normal tissue. The automatic planning features of the planning module 44 may be implemented to support planning of composite ablations, as well as the planning of regular ablation zones achieved using a single applicator, for grid-based and free-hand ablation procedures. When a composite ablation zone is commissioned and used for a given treatment, multiple applicators are to be inserted in the subject's body according the defined placement pattern, and activated simultaneously to achieve the composite ablation zone within the target region. The delivered composite ablation zone resides along an ablation trajectory that lies in-between the entry trajectories used for the insertion of applicators. Usually, this so-called composite ablation trajectory traverses through the center of the composite ablation zone, which coincides with the barycenter of the corresponding applicators. This composite ablation trajectory is oriented parallel to the direction of the corresponding applicators.

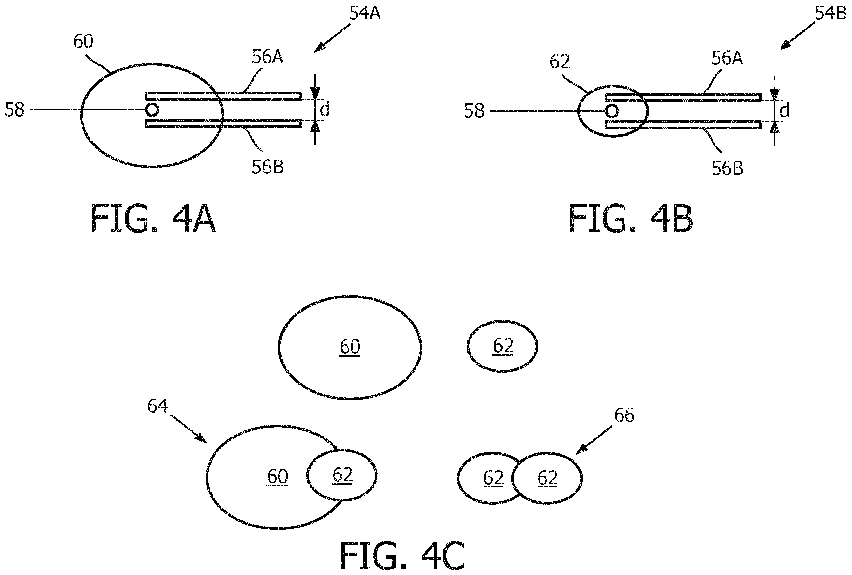

Figs. 4A-4B show some example ablation shapes for a two-applicator linear (placement) pattern, whether determined for a grid or grid-less environment. The applicator pattern and corresponding dimensions may be referenced in a device datasheet and stored, for instance, by the ablation module 38 (Fig. 2). In this particular example, and referring to Fig. 4A, shown is an applicator set 54A (also referred to herein as an applicator pattern) comprising applicators 56A, 56B that define respective vertices or applicator entry trajectories relative to an ablation trajectory 58 for this particular set 54A. Applicator separation dimension, d, may be applicable to the given applicator set, and in one example, may be 1.0 cm. Fig.

4B shows an applicator set (or group) 54B comprising applicators 56A, 56B that define respective vertices or applicator entry trajectories relative to an ablation trajectory 58 for this particular set 54B. Notably, the patterns in Figs. 4A and 4B deliver two different composite ablation zones 60 and 62, respectively, for two different device settings (e.g., power and time ON or duration at the given probe separation dimension). For instance, the ablation size of the composite ablation zone 60 may be based on a higher power level provided via applicator set 54A when compared to the ablation size of the composite ablation zone 62 generated by applicator set 54B, with dimension d having a value of, for instance, 1.0 cm. In other words, applicator sets 54A and 54B may be similarly dimensioned though used at a different power and/or duration level to provide a different composite ablation shape/size.

Fig. 4C shows the resulting composite ablation zones based on differences in settings and also example combinations of composite ablation zones 60 and 62 as selected by the selection module 46 (Fig. 2) to plan ablation therapy treatment. That is, aside from the individual composite ablation zones 60 and 62, additional groups of applicators using the same and/or different placement patterns may be combined by the selection module 46 to provide for further coverage, such as shown with the combined composite ablation zones 64 and 66. Though depicted in Figs. 4A-4C using a combination of simple composite ablation zones 60 and 62 (e.g., shaped respectively as ellipses), the choice of ellipses as a shape is merely for illustration, and as should be appreciated, the group composite ablation zones may be ellipsoidal or non-ellipsoidal (e.g., blended shapes), such as shown in Figs. 1A-1C. In some embodiments, the selection module 46 may take into account the geometrical information about the probe group(s) and the composite ablation zone when selecting a preferred plan (based on selection among the commissioned probes) covering a tumoral region.

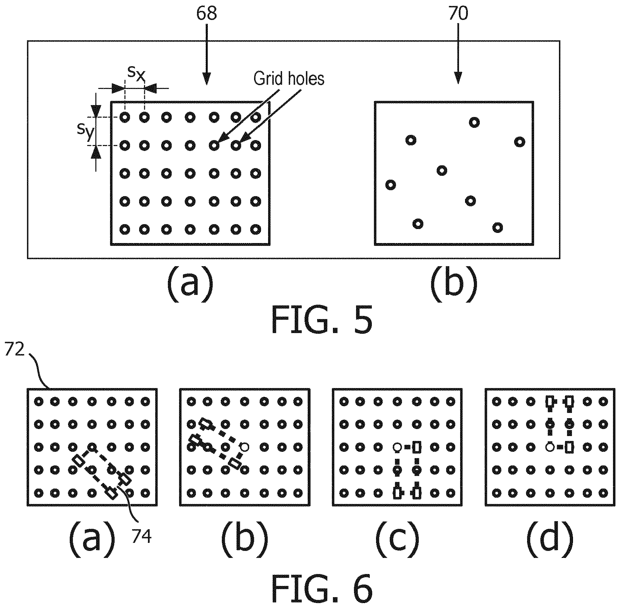

Having described ablation therapy planning in general, attention is now directed specifically to grid-based planning. When an ablation treatment is delivered using a guiding grid template (e.g., a 13x13 brachytherapy grid), a planning algorithm should explore all possible applicator pattern positions matching the grid geometry (e.g., identify the set of all placement patterns for which all applicator positions exactly fit grid holes). However, planning algorithms should also take into consideration that grids may take on various forms. For instance, and referring to Figs. 5(a) and 5(b), shown is a 2D, regular rectangular grid 68 and a 2D irregular grid 70, respectively. In general, in addition to a regular patterned grid with repeated, fixed and regular hole spacing in the x-direction (Sx) and y-direction (Sy), as shown for the grid 68 in Fig. 5(a), some grids may present an irregular layout with grid holes sparsely positioned in space without a fixed spacing in x- and/or y-directions, such as the grid 70 in Fig. 5(b). These differences in grid types may influence the choice of applicator pattern-grid matching method to be used.

The exact matching of the commissioned applicator patterns with the selected grid template is an important prerequisite to allow the actual execution of a final and preferred or optimized treatment plan. Stated otherwise, when applicator vertices of an applicator pattern do not match with grid hole spacing, then the applicator pattern cannot be used for treatment. Figs. 6(a) - 6(d) help to illustrate iterations where matching is, and is not, achieved. In this example, a rectangular applicator pattern 74 is iterated (e.g., advanced and processed according to different positions/orientations) over a grid 72 (also referred to herein as a grid template). That is, the applicator pattern 74 is positioned with one of its vertices matched with a currently processed grid hole (e.g., anchored), and a determination is made as to whether the other vertices (not matched with the currently processed grid hole and hence non-anchored) of the rectangular applicator pattern 74 match the grid holes at each pattern position. In Figs. 6(a) - 6(b), the first two positions for the rectangular applicator pattern 74 in Figs. 6(a) and 6(b) fail to result in matches between the non-anchored vertices and the grid holes, and hence these applicator patterns cannot be used for treatment plan selection (and ultimately, execution). Differently, the rectangular applicator patterns 74 in Figs. 6(c) - 6(d) show that the non-anchored vertices for both pattern positions perfectly match the grid holes and hence these applicator patterns may be selected as potential entry points for treatment plan selection. Indeed, due to the complexity inherent to multiple applicator pattern shapes and dimensions, and to the grid resolution and limitations (e.g., where in some grid templates, such as Civco 14 GA, some column holes are never used and should be neglected during treatment planning), a new set of features is needed for later planning selection (e.g., selection using a set-covering optimization algorithm). Certain embodiments of an applicator-grid pattern matching method seeks to address one or more of these challenges. For instance, given a set of commissioned devices, an applicator-grid pattern matching method may sample all possible applicator pattern positions matching the grid holes. Finally, the set of all possible sampled applicator patterns (e.g., entry points) is provided as input for a subsequent treatment plan selection operation.

To determine the set of all applicator patterns matching a given grid template geometry, certain embodiments of the applicator-grid pattern matching method may take the form of any one of three variations of what may generally be described as a brute force method, including a first method that matches by shifting applicator patterns along all rows and columns of the grid, a second method that matches by discrete local points sets matching, and a third method that matches by continuous local points sets matching. As will be evident from the explanation below, the first and second methods primarily differ in the way applicator patterns and grid holes are represented and processed when matching each other.

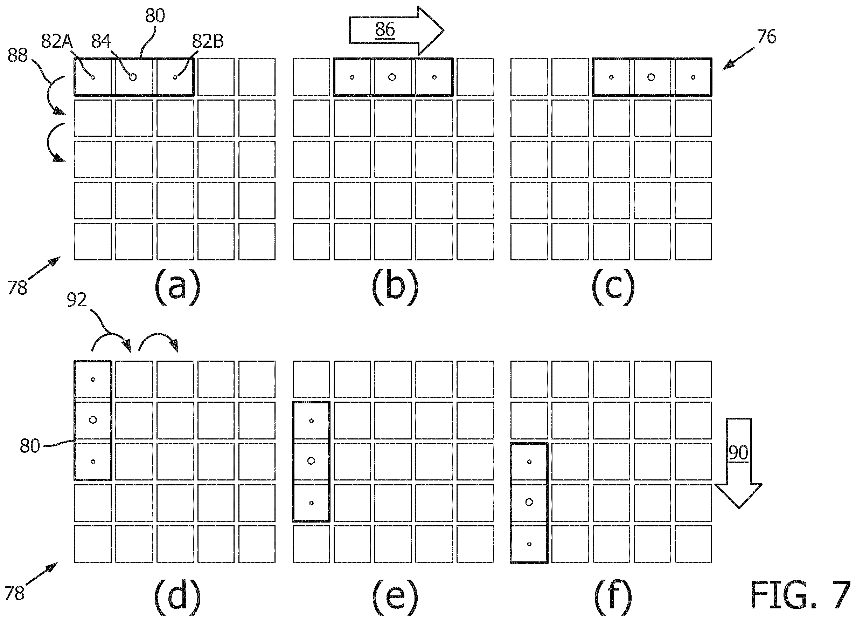

Generally speaking, in a grid scenario, the sampling of groups of entry trajectories is strongly constrained by the regular grid geometry and the grid holes sampling value. For instance, an equilateral triangular pattern cannot be delivered via the grid templates currently supported by some systems, since an equilateral triangle has 60-degree angles, while a square grid has hole-hole diagonals at 45-degree angles. Differently, groups of 2-, 3- or 4-applicators with a linear/segment pattern may be delivered via a grid template if the relative distance of the applicators is a multiple of the grid holes spacing. Figs. 7(a) - 7(f) illustrate one embodiment of a first example applicator-grid pattern matching method (hereinafter, also simply referred to as a first method) 76, which shows a simple and straightforward way to determine placement pattern positions matching the grid holes of a grid or grid template, where the linear patterns match the grid hole spacing dimensions. For instance, and referring to Figs. 7(a) - 7(f), shown are example entry trajectories (and a corresponding ablation trajectory) based on x-direction and/or y-direction shifts for a two-applicator linear pattern in a grid usage scenario. Focusing on Fig. 7(a) for the explanation of features for this diagram, with similar applicability to the other grid depictions in Figs. 7(b) - 7(f), shown is a grid or grid template 78 for which an applicator pattern 80 comprising a pair of linearly-arranged applicators is sampled. The applicator pattern 80 in this example comprises linearly-arranged applicator vertices 82A, 82B corresponding to respective applicator entry trajectories, and the corresponding ablation trajectory 84, the vertices 82A, 82B comprising a linear pattern. The applicator pattern 80 is processed (e.g., sampled) at a given x-y location of the grid 78 for a given row to derive an ablation configuration zone, then iterated (e.g., column-column shifted), for instance, in the x-direction (as denoted by shift symbol 86). The shift 86 may be, in some instances 0.5 centimeters (cm), or whatever grid hole spacing is used, or in some embodiments, a multiple of the grid hole spacing. The applicator pattern 80 is further sampled at this advanced position (Fig. 7(b)), and the process is repeated at the respective advanced positions for that row as shown in Fig. 7(c). This process is repeated for successive rows, as denoted by row-advance symbol 88. In general, for the first method 76, the applicator pattern 80 is initially positioned at the beginning of the first row parallel to the rows-axis and iteratively shifted by the grid hole column spacing till the end of the row is reached. Then, the same pattern is placed at the beginning of the second row and iteratively shifted until the end of the grid for that row is reached, and so on till the last row is processed. In some embodiments, the iterative shifting continuing in the second row may begin at the end of that row, and then when advancing to the third row, the third row iteration starts at the beginning of the third row, and so on where each successive row begins at either the start or end of the successive row according to a somewhat alternating or zig-zag, continuous row advancing pattern. In some embodiments, the iterations may begin at the bottom row (at either end of the grid) and work upward to the top-left position of the grid.

Then, the first method 76 comprises rotating the applicator pattern 80 orthogonally (by ninety-degrees (90°)), and processing continues in similar manner by processing the same applicator pattern 80 along all grid hole columns by shifting the pattern by the grid hole row spacing similar to the method described above and described further in conjunction with Figs. 7(d) - 7(f). Note that in some embodiments, the method 76 may be performed in a different (e.g., reverse) order, such as first according to Figs. 7(d) - 7(f), rotate, and then continue according to Figs. 7(a) - 7(c).

Referring to the grid templates 78 in Figs. 7(d) - 7(f), the applicator pattern 80 is sampled and shifted, as denoted by shift symbol 90, which again may be the grid hole spacing (or in some embodiments, multiples thereof) in the orthogonal direction (e.g., here, in the y-direction) in a similar iterative manner, and likewise, the sampling process continues to be performed for the columns as denoted by column-advance symbol 92. Note that shifts 86 and 90 may be performed according to a difference sequence (e.g., reversed, alternated, etc.) and/or rows and/or columns may be skipped, in some embodiments. The first method 76 automatically computes the applicator patterns 80 that match the grid spacing, and later, single, and/or blended and/or unblended composite ablation zones corresponding to the patterns undergo selection (e.g., using an optimization algorithm). More specifically, at each processed pattern position that matches the grid, the corresponding entry trajectories (e.g., pairs of all applicator pattern vertices positions and the grid plane normal vector) are stored in a general list of entry trajectories and provided as input to the selection module 46 (Fig. 2).

Note that the first method 76 assumes that the relative coordinates of the applicators present in a pattern perfectly fit the grid spacing over the rows and column directions. In the case where the pattern geometry does not match the grid spacing in one or both directions, some pattern positions over the grid are neglected (e.g., omitted from storing), yielding a partially (or even fully) empty list of entry trajectories. Additionally, it is also noted that rotations are limited to ninety-degrees in the first method 76. Also, the first method 76 assumes regular grid geometries, such as the 2D regular rectangular grid 68 shown in Fig. 5(a).

A second example applicator-grid pattern matching method (hereinafter, also simply referred to as a second method) is described below. The second method is more general than the first method 76 since the second method defines a grid to be a set of 2D holes coordinates P:

$$P = \{ \ P_1 \ (x_1, \ y_1), \ P_2 \ (x_2, \ y_2), \ ..., \ P_N \ (x_N, \ y_N) \ \},$$

where N is the total number of grid holes (e.g., N=169 for a brachytherapy grid with 13x13 holes). Similarly, a placement pattern is defined as a set V of pattern vertices' 2D relative coordinates with respect to the pattern center's position:

$$V = \{ \ V_1 \ (x_1, \ y_1), \ ..., \ V_L \ (x_L, \ y_L) \ \},$$

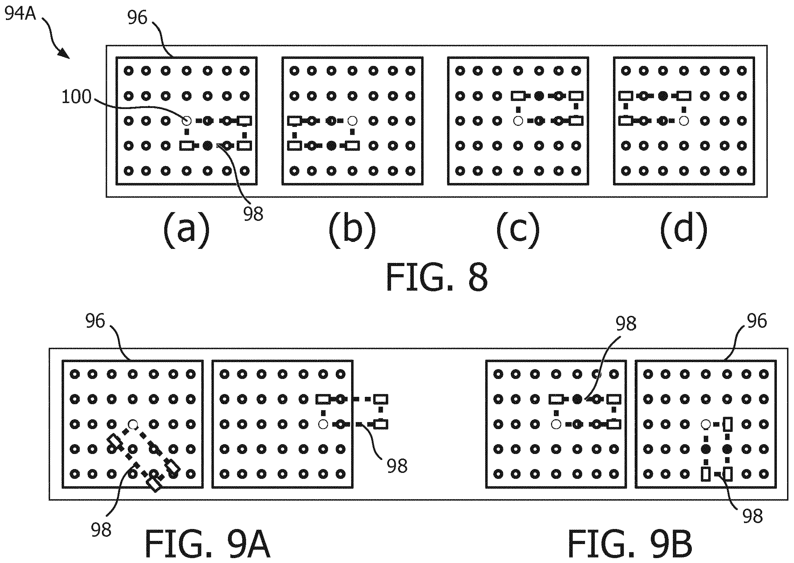

where L represents the total number of applicator pattern vertices (e.g., L = 3 for a triangular pattern with three applicators correspondingly placed along the three vertices). Compared to the first method 76, for the second method, no other assumptions are (strictly) maintained over the grid and the applicator pattern geometry. Referring to Figs. 8(a) - 8(d), shown is a simple illustration of the second method, denoted second method 94a, using a 2D regular rectangular grid 96 and an applicator pattern 98 in the form of a rectangular pattern having four vertices, to use an illustrative example. In this example, the applicator pattern 98 has one of its vertices (top left) anchored to a currently processed grid hole 100 in Fig. 8(a), with the anchored vertice shown in the figures as a darkened, filled dot and the non-anchored vertices are shown as white-filled squares. In Fig. 8(b), the applicator pattern 98 is shifted left so that the other top vertice (top right) is now matched with the currently processed grid hole 100, now becoming the anchored vertice. In Fig. 8(c), the applicator pattern 98 shown in Fig. 8(a) is shifted upwards so that another vertice (bottom left) is matched with the currently processed grid hole 100, and is now the anchored vertice. Then in Fig. 8(d), the applicator pattern 98 shown in Fig. 8(c) is shifted left so that the bottom right vertice is matched with the currently processed grid hole 100 and now becomes the anchored vertice. Note that no particular order in the shifts is assumed by this example for all cases, and that in some embodiments, shifts in a different order may be performed to successively anchor each vertice of the applicator pattern 98 to the currently processed grid hole 100. Each pattern position/orientation shown in Figs. 8(a) - 8(d) is accepted and stored, since all non-anchored pattern vertices for each position match a grid hole. In the example second method 94a, in general, a given applicator pattern is iteratively anchored via each of its vertices $V_i$ in the set V to each point $P_j$ in the set P. In one embodiment, an anchored applicator pattern to a point $P_j$ is accepted and collected in a suitable pattern list, if and only if, all L pattern vertices present in V are also present in the set P (i.e., $V \subseteq P$, all pattern vertices are also grid holes). Fig. 9A shows two examples where positioning of the applicator pattern 98 on the grid 96 is rejected, since not all of the vertices for each of the depicted pattern placements are matched to grid holes. On the other hand, Fig. 9B shows two examples where positioning of the applicator pattern 98 on the grid 96 is accepted and stored (collected), since all of the vertices for each of the depicted pattern placements are matched to grid holes.

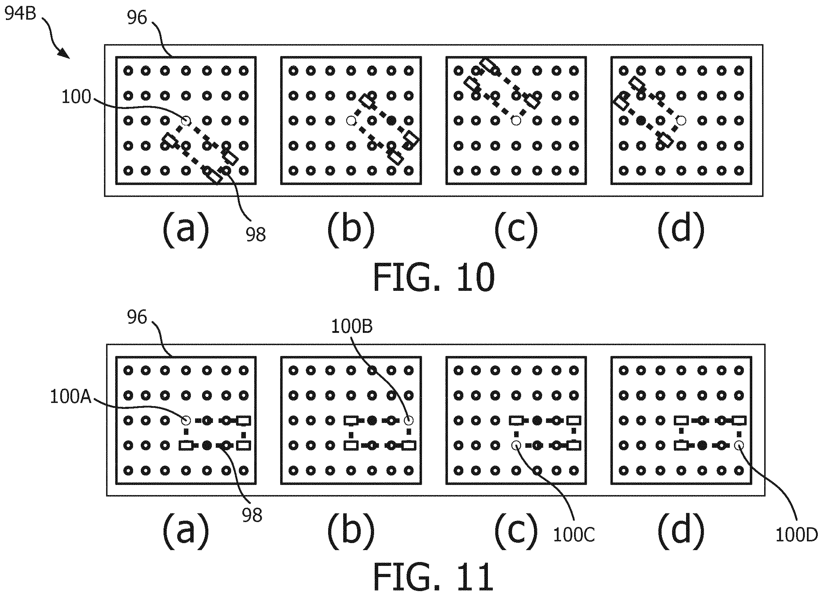

The second method 94a may optionally be extended to include stepped angular rotations as part of the matching process. Referring to Figs. 10(a) - 10(d), shown is an example second method 94b, which illustrates the stepped angular rotations. Shown is the grid 96, the applicator pattern 98, and the currently processed grid hole 100 where each vertice of the applicator pattern 98 is iteratively anchored. In this example, similar to Fig. 8(a), the applicator pattern 98 has one of its vertices (top left, when the applicator pattern is viewed in a horizontal orientation parallel to the longest axis of the applicator pattern 98) anchored to a currently processed grid hole 100 in Fig. 10(a), yet rotated a defined angular step. In other words, for the currently processed grid hole 100 and for each vertice, matching may be performed according to the shifts in the applicator pattern 98 described above for Figs. 8(a) - 8(d), and also for a defined rotation. In Fig. 10(b), for the given angular rotation, the applicator pattern 98 is shifted up and to the right so that the lower left vertice (again, from the perspective of when the applicator pattern 98 is situated horizontally) is now matched with the currently processed grid hole 100, now becoming the anchored vertice. In Figs. 10(c) - 10(d), the applicator pattern 98 is shifted, for the given angular rotation, so that the lower right vertice is the anchored vertice and the upper right vertice (using the same perspective of the horizontal orientation for the pattern as described above) is the anchored vertice, respectively. Note that in this example, none of the patterns would be accepted since the non-anchored vertices do not match with the grid holes. Note also that the order of shifts may differ from those depicted in the examples of Figs. 10(a) - 10(d). In one embodiment, the applicator pattern 98 is rigidly rotated by different angular steps, $\gamma$, to verify and match all possible discretely rotated pattern positions and orientations over the grid plane. The angular step may be pre-configured, or user-configured (e.g., via a configuration menu). For smaller step values, a slower iterative matching search is performed, though with possibly higher matching accuracy (and vice versa, where larger step values may result in faster performance with less relative accuracy). A pattern rotation may be achieved by simply multiplying all coordinates of the pattern vertices in the set V by a basic 2D rotation matrix:

$$V_{rot\gamma}(x, y) = \begin{bmatrix} cos\gamma & -sin\gamma \\ sin\gamma & cos\gamma \end{bmatrix} * V(x, y).$$

Note that an embodiment of a second method that comprises the collective functionality of second methods 94a and 94b may be referred to as second method 94.

In some embodiments, when matching pattern vertices in V and grid holes positions in P, some distance tolerance, epsilon or $\varepsilon$, may be provided and used by the second method algorithm to accept and also collect patterns that are not exactly positioned at grid holes, yet they are still at a distance smaller than a given tolerance. This feature may be beneficial in clinical scenarios where clinical experts accept some inaccuracy in the placement of applicators via the grid. Explaining further, the tolerance $\varepsilon$ is intended to permit selection of an insertion pattern even in cases where the distance between grid holes and pattern vertices is smaller than a given epsilon, $\varepsilon$. This tolerance provides more freedom to the matching search. Exact execution of the returned insertion positions may not be possible (e.g., the finally positioned applicators' pattern via the grid holes may slightly differ from what is shown on the device datasheet). However, the use of the tolerance assumes a clinical specialist is taking the responsibility to allow a minor deviation from datasheet information via adding a tolerance of a certain $\varepsilon$ (e.g., in x mm).

In some embodiments, to avoid redundant computations, during the iterative matching search of the second method, positioned patterns that have already been anchored and processed at current and previous points in P are neglected. For instance, and referring to Figs. 11(a) - 11(d), shown are applicator patterns 98 where, despite the change in grid hole corresponding to the anchored vertice of the applicator pattern 98, the same grid holes are matched in the search. For instance, in Fig. 11(a), the processed grid hole 100a is centered in the grid 96, with the top left vertice of the applicator pattern anchored there. In Fig. 11(b), the processed grid hole 100 is located where the top right non-anchored vertice of the applicator pattern 98 of Fig. 11(a) was positioned, yet the same grid holes are matched with the vertices of the applicator pattern 98. Thus, in certain embodiments, the second method skips or neglects (e.g., omits) the collection or storage of the applicator pattern 98 in Fig. 11(b). Similarly, Figs. 11(c) and 11(d) show the anchored vertice to be where the non-anchored, lower left and the non-anchored lower right vertices of the applicator pattern 98 in Fig. 11(a) were located, and so these applicator patterns 98 of Figs. 11(c) - 11(d) are skipped or neglected from storage, as they, like the applicator pattern 98 of Fig. 11(b), are redundant to the applicator pattern 98 of Fig. 11(a).

Note that in some embodiments of the second method 94, in certain instances where a 2D rigid regular grid (e.g., a brachytherapy grid template) with fixed spacing $S_x$ and $S_y$ along grid columns and rows is used for treatment, this additional geometrical information may be exploited by the second method matching algorithm to filter out applicator patterns that would never fit the grid geometry. For instance, a pattern-grid geometry check may be performed by computing all relative distances between all pairs of vertices at set V along the x and y grid plane axes. If all computed distances are equal to (or multiples of) the corresponding $S_x$ and $S_y$ spacings, then the current applicator pattern may potentially match the grid holes and may be further processed to retrieve (all) possible feasible positions over the grid, otherwise the currently processed applicator pattern may simply be neglected (not stored).

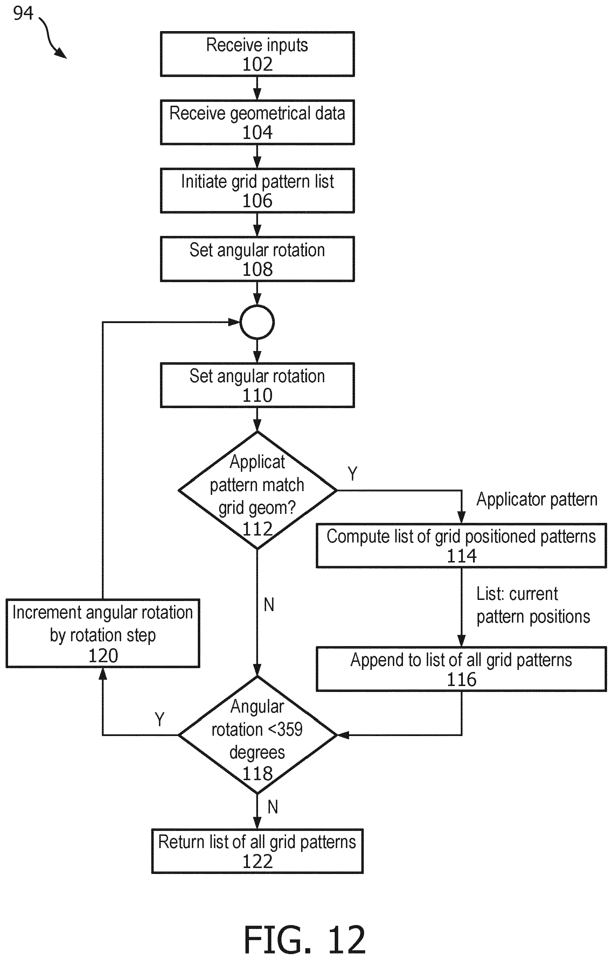

Reference is now made to Fig. 12, which is a flow diagram that illustrates an embodiment of the example second method 94. The method 94 receives inputs in step 102. For instance, the method 94 receives plural objects, including the grid, applicator pattern, and rotation step. These objects may be received from local or remote storage, such as via a GET command. In step 104, the method 94 receives geometrical data of the grid from the grid object. Again, the receipt may be achieved via a GET command. Though steps 102 and 104 are described using GET commands, other existing mechanisms and/or programming languages may be used to receive the information and/or perform similar functionality. The method 94 further comprises initiating a list of all grid patterns in step 106. For instance, the initialization is of the returned list of matching insertion patterns found during iterations. In step 108, the method 94 sets the angular rotation ($\gamma$) to zero.

In step 110, the method 94 rotates the applicator pattern by the angular rotation (e.g., in the first pass, the angular rotation is zero). In step 112, the method 94 determines if the applicator pattern matches the grid geometry. For instance, in step 112, the method 94 performs a pattern-grid geometry check that may be implemented by computing all relative distances between all pairs of vertices at set V along the x and y grid plane axes. If all computed distances are equal to (or in some embodiments, a multiple of) the corresponding $S_x$ and $S_y$ spacings, then the current pattern may potentially match the grid holes and can be further processed to retrieve a plural or all possible feasible positions over the grid (otherwise, the currently processed pattern can be simply neglected). In other words, if yes to step 112, the method 94 proceeds to step 114, where the method computes the list of all grid-positioned patterns. As explained above, in one embodiment, the method 94 matches the applicator pattern over all grid holes, and all matching patterns are added to a list. It is noted that the flow diagram in Fig. 12 shows an outer loop, which iteratively increases the $\gamma$ (angular rotation) and appends newly found patterns to the returned list. The flow diagram also shows an inner loop (e.g., within step 114), where the method 94 computes the total list of the currently rotated application pattern by iteratively shifting and anchoring it over all grid holes (similar to the process performed by the first method 76). In other words, the outer loop increases the angular rotation, $\gamma$, by a step, and the inner loop (in step 114) shifts this rotated pattern over all grid holes.

In step 116, the method 94 appends the list of current pattern positions (that match the grid holes) from step 114 into a list of all grid patterns. In step 118, the method 94 determines whether the angular rotation is less than three hundred, fifty-nine degrees (359°). Note that step 118 may be reached when the determination in step 112 is in the negative (e.g., no). If the angular rotation is less than three hundred, fifty-nine degrees (359°), the method proceeds to step 120, where the angular rotation is adjusted by a rotation step (the rotation step, in degrees, used to rotate an insertion pattern). In one embodiment, the rotation step is user-defined. From there, the loop continues (e.g., at step 110). If the angular rotation is not less than three hundred, fifty-nine degrees (359°) in step 118, the method proceeds to step 122, where a list of all grid-applicator patterns that are matching is returned. As similarly done for the first method 76, the returned list (e.g., of all applicator patterns matching the grid) is provided as input to the selection module 46 (Fig. 2).

Note that, as with the get command, the use of a return command is suggested yet merely illustrative of a function in the process using an example programming language, and that in some embodiments, other existing mechanisms (e.g., using other programming languages) may be used to perform similar functionality. In some embodiments, certain steps may be combined, or otherwise altered to perform similar functionality.

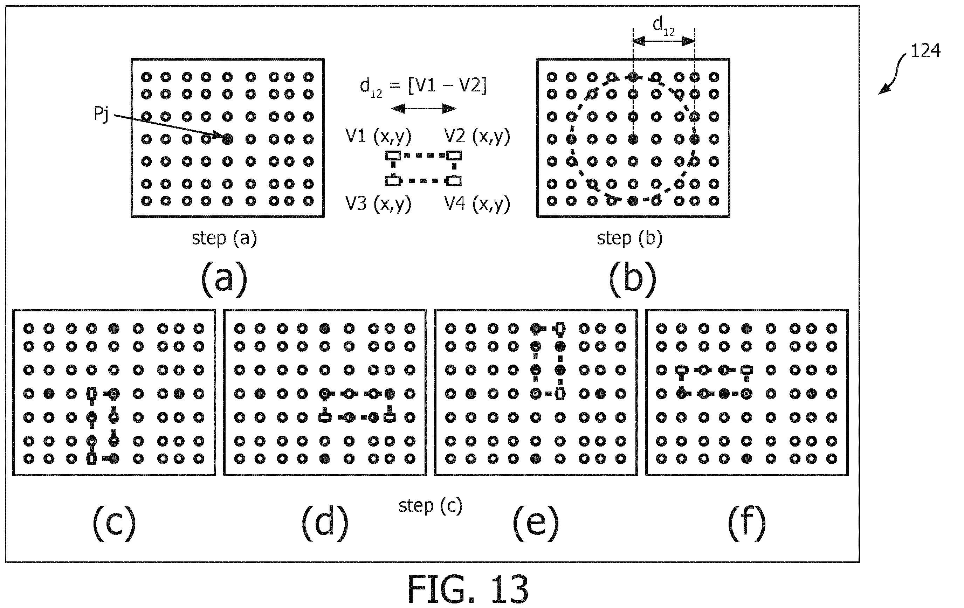

Having described a first method 76 and a second method 94, attention is now drawn to a third example applicator-grid pattern matching method (hereinafter, also simply referred to as a third method) where matching is performed using continuous local points sets matching. The third method may be considered a modified version of the second method, where a continuous applicator pattern rotation is implicitly performed during a (brute-force) iterative grid-pattern matching search. That is, no angular steps in $\gamma$ are needed to verify and match all possible rotated pattern positions and orientations over the grid plane. Similar to the second method, for the third method, a grid is also defined as a set of 2D holes coordinates P, and the applicator pattern comprises a set V of 2D pattern vertices' 2D relative coordinates with respect to the pattern center position. In this third, iterative matching algorithm, iterations are performed over all grid points $P_j$ in the set P. At each j-th iteration the first applicator pattern vertex $V_1$ is positioned at the currently processed grid point, $P_j$ (as further illustrated in Fig. 13), then the algorithm proceeds according to different conditions as described in the following.

If L = 1 (e.g., the applicator pattern contains only a single vertex, or, that is, a single applicator delivering a single, non-composite ablation trajectory or zone), the current applicator pattern at position $V_1 = P_j$ is collected in a list of selected grid matching applicator patterns and the algorithm proceeds to the next iteration and to the next grid point in the set P. In this simple case where the total number of applicator pattern vertices (i.e., L) is equal to 1, the third method shifts a point over all grid holes. This is an extreme case of a scenario covering standard treatment delivery using only non-composite ablation shapes (e.g., standard ellipsoidal ablation zones).

For $L \geq 2$, reference is made to the third method, denoted 124 in Figs. 13(a) - 13(f), where Fig. 13(a) illustrates step (a) of the method 124, Fig. 13(b) illustrates step (b) of the method 124, and Figs. 13(c) - 13(f) are used to illustrate step (c) of the method 124. For L = 2, after having set the first applicator pattern vertex $V_1$ at the currently processed grid point $P_j$ (step (a), Fig. 13(a)), the distance $d_{12}$ between pattern vertices $V_1$ and $V_2$ is computed (as illustrated by the top schematic between steps (a) and (b)), and a search for all grid points in the set P at a distance $d_{12}$ from $P_j$ is performed. Here, the subset of found grid points is defined as $S_j$ (e.g., see step (b), Fig. 13(b), where the subset $S_j$ includes the grid points along the dotted circle at the prescribed distance, which in this example includes four grid points at the 12:00, 3:00, 6:00, and 9:00 o'clock locations on the dotted circle). If the cardinality of the set $|S_j| = 0$ (i.e., no grid points have been found in the set P at a distance $d_{12}$ from $P_j$), the current *j*-th iteration is stopped and the algorithm proceeds to the next iteration. While if $|S_j| = n_s > 0$, all $n_s$ combinations of applicator patterns, with $V_1$ positioned at $P_j$ and $V_2$ positioned alternatively to each grid point in the set $S_j$, are collected in the list of selected grid matching patterns. Finally, the algorithm proceeds to the next iteration and to the next grid point in the set P. In effect, the third method for L = 2 is searching at all angles within a continuous space.

If L > 2 and $|S_j| = n_s > 0$, a set of $n_s$ potential applicator patterns' positions are determined by positioning $V_1$ at grid point $P_j$ and $V_2$ positioned alternately to each of the s grid points in the set $S_j$ (see step (b), Fig. 13(b)). For each of these s pairs of pattern vertices' grid positions $(V_1, V_2)$, all remaining (L - 2) applicator pattern vertices' positions are easily retrieved using the pattern relative coordinates in the set V (see Figs. 13(c) - 13(f)). Each of these $n_s$ positioned applicator patterns is included in the list of selected grid matching patterns, if and only if, also the remaining (L - 2) pattern vertices are present in the set P (i.e., $V \subseteq P$, all pattern vertices are also grid holes). Finally, the algorithm proceeds to the next iteration and to the next grid point in the set P.

Explaining further, Figs. 13(a) - 13(f) use a rectangular applicator pattern (shown in the lower schematic between step (a) and step (b)) for demonstrating an iterative algorithm for the third method for the case where L > 2. In Fig. 13(a), step (a) of the iterative algorithm is shown, where the first pattern vertex $V_1$ is positioned at the currently processed grid point $P_j$. In Fig. 13(b), step (b) of the iterative algorithm is shown, where the subset $S_j$ consists of 4 grid points at a distance $d_{12} = |V_1 - V_2|$ from the processed grid point $P_j$. In Figs. 13(c) - 13(f), the step (c) of the iterative algorithm is shown, where the set of 4 potential applicator patterns' positions are retrieved and shown by positioning $V_1$ at grid point $P_j$ (step (a)) and $V_2$ positioned alternately to each of the 4 grid points in the set $S_j$ (step (b)), and for each of these 4 pairs of pattern vertices' grid positions $(V_1, V_2)$, the remaining two applicator pattern vertices' positions $V_3$ and $V_4$ (white squares) are easily retrieved using the pattern relative coordinates in the set V. Each of these 4 positioned applicator patterns is included in the list of selected grid matching patterns, if and only if, also the remaining two pattern vertices $V_3$ and $V_4$ are present in the set P (i.e., $V \subseteq P$, all pattern vertices are also grid holes).

Note that certain features described above for the second method likewise apply to the third method. For instance, for the third method, a distance tolerance $\varepsilon$ may be provided and used by the algorithm when matching pattern vertices in V and grid holes positions in P to accept and collect also patterns that are not exactly positioned at grid holes, but are still at a distance smaller than the given tolerance. Likewise, to avoid any redundant computation during the iterative matching search, all computed applicator patterns that were already processed at previous iterations, or that have some of their vertices outside of the field of view of the grid plane, are neglected.

Finally, the list of all selected applicator patterns that match the grid in combination with the grid plane normal vector are used to populate the list of all groups of entry trajectories used as input to the selection module 46 (Fig. 2). Explaining further, once a grid is positioned by the clinical expert, it may be perfectly represented in 3D space by a local coordinate system where the 3D guiding grid plane's x-y axes are typically taken parallel to the two longer edges of the grid, and the z-axis is orthogonal to the x-y plane and parallel to the third shorter grid edge (i.e., the grid plane normal vector).

The z-axis it is also typically pointed from outside to the inner part of the subject's body (i.e., it is pointing towards the tumoral region of interest). In one embodiment, the algorithm automatically computes a set of applicator patterns' positions, where these positions are the matching grid holes. These grid holes are just 3D points on the grid x-y plane. To represent a "trajectory" in 3D space, one point (i.e., the trajectory origin) and one direction vector is needed. Accordingly, the list of all groups of entry trajectories are obtained by adding the grid plane normal vector (i.e. the trajectory direction vector) to the automatically computed applicator patterns' entry points (i.e., trajectory origins).

Though largely described in the context of composite ablations, it should be appreciated that all three methods (first, second, and third) also support, as a special case, standard, single virtual ablation zones delivered via a single applicator. In such instances, such as for instance the second and third method, the pattern vector V contains only a single element, and the iterative algorithm returns, as a result, the list of entry trajectories orthogonal to the grid plane and originating from all grid holes.

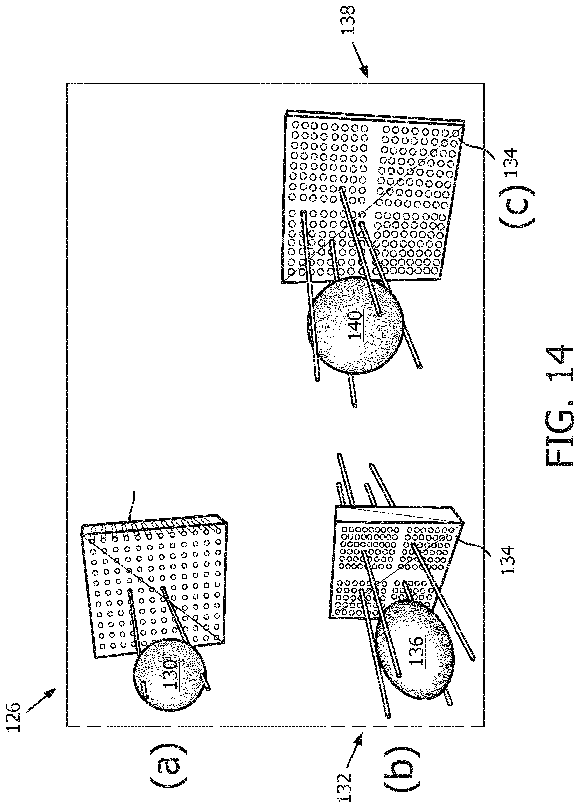

Referring now to Figs. 14(a) - 14(c), shown are some examples of composite ablation zones delivered via linear and square applicators' patterns and generated using the grid-pattern matching approach of the third method. Composite zones generated by multiple applicators inserted via selected insertion patterns may be of different shapes. Though it should be appreciated that composite zones may be simulated using blended shapes, in the simulated examples depicted in Figs. 14(a) - 14(c), a simple ellipsoid (with no blending, such as via implicit functions as described above) is used here for illustration. In Fig. 14(a), an applicator pattern 126 comprises two linearly-arranged applicators inserted into a 13 x 13 regular brachytherapy grid 128. The applicator pattern 126 delivers a simulated composite (ellipsoid) ablation zone 130. In Fig. 14(b), an applicator pattern 132 comprises four applicators arranged in a rectangular pattern, the applicator pattern 132 inserted in a grid 134 made up of 21x21 holes with removal of the mid row and column. The applicator pattern 132 delivers the simulated composite ablation zone 136. Similarly, in Fig. 14(c), the applicator pattern 138 comprises four applicators that are inserted differently in the grid 134 (than in Fig. 14(b)), and deliver the simulated composite ablation zone 140 as shown. Each of these simulations are the result of the grid-pattern matching, third method identifying the set of all placement patterns for which all applicator positions fit the grid.

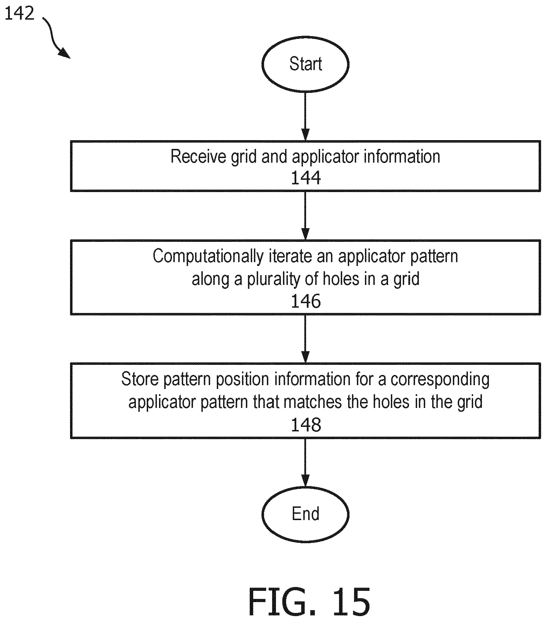

In view of the description above, it should be appreciated that one embodiment of an applicator-grid pattern matching method that computes (e.g., using in one embodiment the computing device 14, Fig. 2) applicator insertion patterns for use in grid-based ablation, and which generally describes the three methods, is denoted as method 142 in Fig. 15. The method 142 comprises receiving grid and applicator information (144); computationally iterating an applicator pattern along a plurality of holes in a grid, wherein computationally iterating comprises advancing an applicator pattern object throughout a grid object, wherein the applicator pattern is defined by one or more vertices corresponding to one or more applicator entry trajectories, and an ablation trajectory (146); and storing pattern position information for a corresponding applicator pattern that matches the holes in the grid, wherein the pattern position information comprises an entry trajectory comprising applicator pattern vertice position or positions and a grid plane normal vector (148).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. Note that various combinations of the disclosed embodiments may be used, and hence reference to an embodiment or one embodiment is not meant to exclude features from that embodiment from use with features from other embodiments. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical medium or solid-state medium supplied together with or as part of other hardware, but may also be distributed in other forms. Any reference signs in the claims should be not construed as limiting the scope.

**Claims**

1. A method (142) for computing applicator insertion patterns for use in grid-based ablation, the method, comprising:

    at a computing device (14):
    receiving grid and applicator information (144);
    computationally iterating an applicator pattern along a plurality of holes in a grid, wherein computationally iterating comprises advancing an applicator pattern object throughout a grid object, wherein the applicator pattern is defined by one or more vertices corresponding to one or more applicator entry trajectories, and an ablation trajectory (146); and
    storing pattern position information for a corresponding applicator pattern that matches the holes in the grid, wherein the pattern position information comprises an entry trajectory comprising applicator pattern vertice position or positions and a grid plane normal vector (148).

2. The method of the previous claim, wherein computationally iterating comprises iteratively advancing, by one hole in a first direction followed by one hole in an orthogonal, second direction, the applicator pattern until all possible applicator patterns in the first and second directions have been attempted (76).

3. The method of any one of the previous claims, wherein the first direction is parallel to a row or column.

4. The method of any one of the previous claims, wherein computationally iterating comprises, for each hole of the grid:

   successively anchoring each of the vertices of the applicator pattern to the each hole of the grid;
   for each applicator pattern position relative to the anchored vertice, determining if all other vertices of the applicator pattern match a respective hole of the grid, wherein the storing the pattern position information comprises storing the pattern position information for each of the applicator pattern positions corresponding to the anchored vertice where the other vertices match the respective holes of the grid; and
   rejecting applicator pattern positions for a given anchored position where at least one of the vertices does not match a grid hole (94).

5. The method of any one of the previous claims, further comprising rotating the applicator pattern a defined angular step and repeating the successively anchoring, determining, storing, and rejecting.

6. The method of any one of the previous claims, further comprising applying a distance tolerance when determining the match between the vertices and the holes.

7. The method of any one of the previous claims, further comprising omitting, from storing, redundant pattern position information or pattern position information for applicator patterns for which at least one of the vertices is outside of the grid.

8. The method of any one of the previous claims, wherein the applicator pattern consists of two vertices, and for a given hole coinciding with one of the two vertices, the computationally iterating comprises:

   determining a distance between the two vertices;
   searching for all holes at the distance from the given hole, wherein storing the pattern position information comprises storing pattern position information for all applicator patterns where the two vertices match holes of the grid located at the distance; and
   repeating the determining and the search for successive holes (124).

9. The method of any one of the previous claims, wherein the applicator pattern comprises more than two vertices, and for a given hole coinciding with one of the two vertices, the computationally iterating comprises:

   determining a distance between the two vertices;
   searching for all holes at the distance from the given hole and defining each pair of the vertices along the searched distance that match with the holes as a set;
   for each set, determining remaining applicator pattern vertices, wherein storing the pattern position information comprises storing pattern position information for each set if remaining vertices of the applicator pattern for each set also match holes of the grid; and
   repeating the determining, searching, and determining for successive holes (124).

10. The method of any one of the previous claims, further comprising applying a distance tolerance when determining the match between the vertices and the holes.

11. The method of any one of the previous claims, further comprising omitting, from storing, redundant pattern position information or pattern position information for applicator patterns for which at least one of the vertices is outside of the grid.

12. The method of any one of the previous claims, further comprising providing the applicator patterns and pattern position information to a planning algorithm for selection of applicator patterns for use in an ablation process.

**13.** The method of any one of the previous claims, wherein for a single vertice, the pattern position information comprises a list of entry trajectories corresponding to all holes of the grid.

**14.** A computing device (14) for implementing the any one of the preceding method claims.

**15.** A non-transitory, computer readable storage medium (20) comprising instructions that when executed by the one or more processors, causes the one or more processors to implemented the method of any one of the preceding claims.

10A

FIG. 1A

10B

FIG. 1B

10C

FIG. 1C

12

| UI 24 | Imaging 26 | Ablation 28 |

Computing device 14

I/O 18

P 16A --- P 16N

DBUS 22

MEM 20

OS 30

STOR DEV

Ablation therapy software 32

| GUI 34 | Commissioning 40 |

Planning 44

AGPM 42

Segmentation 36

Selection 46

| Ablation 38 | Guidance 48 |

# FIG. 2

FIG. 3

FIG. 4A

FIG. 4B

FIG. 4C

EP 4 501 266 A1

FIG. 5

FIG. 6

EP 4 501 266 A1

FIG. 7

FIG. 8

FIG. 9A

FIG. 9B

FIG. 10

FIG. 11

94

```
┌─────────────────────────┐
│     Receive inputs      │
│          102            │
└─────────────────────────┘
             │
             ▼
┌─────────────────────────┐
│ Receive geometrical data│
│          104            │
└─────────────────────────┘
             │
             ▼
┌─────────────────────────┐
│  Initiate grid pattern list │
│          106            │
└─────────────────────────┘
             │
             ▼
┌─────────────────────────┐
│   Set angular rotation  │
│          108            │
└─────────────────────────┘
             │
             ▼
            ( )
             │
             ▼
┌─────────────────────────┐
│   Set angular rotation  │
│          110            │
└─────────────────────────┘
             │
             ▼
         ◇ Applicat
         pattern match      Y
         grid geom?  ─────────────►  Applicator pattern
           112
             │ N                ┌──────────────────────────────────────┐
                                │ Compute list of grid positioned patterns │
                                │              114                        │
                                └──────────────────────────────────────┘
                                              │  List: current
                                              │  pattern positions
┌──────────────────────────┐                  ▼
│ Increment angular rotation│     ┌──────────────────────────────────────┐
│    by rotation step       │     │  Append to list of all grid patterns  │
│          120              │     │              116                       │
└──────────────────────────┘     └──────────────────────────────────────┘
             ▲                                │
             │ Y        ◇ Angular             │
             └──────── rotation <359 ◄────────┘
                        degrees
                          118
                           │ N
                           ▼
              ┌─────────────────────────┐
              │ Return list of all grid patterns │
              │          122            │
              └─────────────────────────┘
```

# FIG. 12

FIG. 13

FIG. 14

142

```
        ( Start )
           |
           v
+----------------------------------------+
| Receive grid and applicator information|
|                  144                   |
+----------------------------------------+
           |
           v
+----------------------------------------+
|  Computationally iterate an applicator |
|    pattern along a plurality of holes  |
|              in a grid                 |
|                  146                   |
+----------------------------------------+
           |
           v
+----------------------------------------+
| Store pattern position information for |
|   a corresponding applicator pattern   |
|  that matches the holes in the grid    |
|                  148                   |
+----------------------------------------+
           |
           v
        (  End  )
```

# FIG. 15

Europäisches
Patentamt
European
Patent Office
Office européen
des brevets

# EUROPEAN SEARCH REPORT

Application Number

EP 23 19 9022

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 190 252 A1 (KONINKLIJKE PHILIPS NV [NL]) 7 June 2023 (2023-06-07) | 1-3,6,7, 10-15 | INV. A61B34/10 |
| A | * paragraphs [0051], [0083], [0087] * ----- | 4,5,8,9 | A61B17/34 A61B90/00 |
| A | EP 3 777 748 A1 (KONINKLIJKE PHILIPS NV [NL]) 17 February 2021 (2021-02-17) * paragraphs [0142], [0161], [0162] * ----- | 1 | A61B18/12 |
| A | US 2019/008591 A1 (DESAI DEVASHREE S [US] ET AL) 10 January 2019 (2019-01-10) * paragraphs [0077], [0085] - [0087] * ----- | 1 | |

TECHNICAL FIELDS
SEARCHED     (IPC)

A61B

The present search report has been drawn up for all claims

| Place of search | Date of completion of the search | Examiner |
|---|---|---|
| Munich | 28 February 2024 | Angeli, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 9022

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2024

| Patent document cited in search report | | Publication date | Patent family member(s) | | Publication date |
|---|---|---|---|---|---|
| EP 4190252 | A1 | 07-06-2023 | EP | 4190252 A1 | 07-06-2023 |
| | | | WO | 2023099299 A1 | 08-06-2023 |
| EP 3777748 | A1 | 17-02-2021 | CN | 114302687 A | 08-04-2022 |
| | | | EP | 3777748 A1 | 17-02-2021 |
| | | | EP | 4013332 A1 | 22-06-2022 |
| | | | US | 2022265359 A1 | 25-08-2022 |
| | | | WO | 2021032449 A1 | 25-02-2021 |
| US 2019008591 | A1 | 10-01-2019 | EP | 3648692 A1 | 13-05-2020 |
| | | | JP | 6959428 B2 | 02-11-2021 |
| | | | JP | 7289340 B2 | 09-06-2023 |
| | | | JP | 2020526290 A | 31-08-2020 |
| | | | JP | 2022002745 A | 11-01-2022 |
| | | | US | 2019008591 A1 | 10-01-2019 |
| | | | US | 2020281658 A1 | 10-09-2020 |
| | | | WO | 2019010232 A1 | 10-01-2019 |

EPO FORM P0459

For more details about this annex : see Official Journal of the European Patent Office, No. 12/82